Europäisches Patentamt

European Patent Office

Office européen des brevets

(11) Publication number: **0 372 596**
A2

# EUROPEAN PATENT APPLICATION

(21) Application number: 89202745.9

(51) Int. Cl.⁵: **A21D 13/08**

(22) Date of filing: 31.10.89

(30) Priority: 07.11.88 US 268308

(43) Date of publication of application:
13.06.90 Bulletin 90/24

(84) Designated Contracting States:
AT BE CH DE ES FR GB GR IT LI LU NL SE

(71) Applicant: THE PROCTER & GAMBLE
COMPANY
One Procter & Gamble Plaza
Cincinnati Ohio 45202(US)

(72) Inventor: Cappel, James Wilbur
4837 Potomac Drive
Fairfield Ohio 45014(US)
Inventor: Prosise, Robert Lawrence
7104 Larchwood
Cincinnati Ohio 45241(US)
Inventor: Rece, Robert Daniel
914 Fawn Hill Drive
Edgewood, KY 41017(US)
Inventor: Berry, Delia Ann
3538 Linwood Avenue
Cincinnati Ohio 45226(US)

(74) Representative: Canonici, Jean-Jacques et al
Procter & Gamble European Technical
Center N.V. Temselaan 100
B-1820 Strombeek-Bever(BE)

(54) Cookies made with low Aw fibercontaining fillings.

(57) The invention relates to cookies made with a fiber-containing filling. This filling has a very low water activity (0.2-0.35) so that the crispness of the cookies' outer dough is preserved. At the same time, the filling tastes soft and lubricious instead of tough and fibrous. In particular, the invention is a dual textured cookie made from: (a) discrete regions of a cookie dough which before baking contains: 10% to 30% shortening, 20% to 45% flour, 20% to 45% readily crystallizable sugar, and 5% to 15% water; and (b) discrete regions of a filling which before baking contains: 0% to 25% sorbitol, 10% to 45% fructose, 0% to 30% dextrose, 16% to 32% glycerine, 2% to 35% cellulosic fiber, and 3% to 10% fruit puree.

## COOKIES MADE WITH LOW $A_w$ FIBER-CONTAINING FILLINGS

### Technical Field

The invention relates to the field of cookies, and in particular cookies having a crisp outer surface and a soft-textured inner filling containing fiber.

### Background of the Invention

The present development relates to cookies that contain fillings made with fiber. Most conventional cookies with fillings have either a soft-textured outer dough with a medium water activity filling, or a crisp outer dough with a tacky, tough low water activity filling. For example, fig bars have a soft outer dough and medium water activity filling. Certain kinds of dietary cookies have a crisp outer dough and a low $A_w$ filling, but the filling has a tough, fibrous texture.

The cookies of the present invention, on the other hand, have a crisp outer dough and a low $A_w$ filling, and the filling is soft, lubricious and great-tasting instead of tough and fibrous. Moreover, the water activity of the filling is low enough to preserve the crispness of the outer dough.

Various food compositions made with fiber are known to the art. For example, U.S. Patent 4,698,232 to Sheu et al., issued October 6, 1987, discloses a fiber-containing confectionery composition said to have a soft, non-fibrous texture and smooth mouthfeel. The composition is made with: (a) a fiber pre-treated with fat and glycerine; (b) gelatin, gum arabic, and high fructose corn syrup; and (c) a mixture of corn syrup and high fructose corn syrup, and optionally a sweetener such as dextrose, fructose, or sorbitol. The coating of fat and glycerine on the fiber particles is said to provide a lubricating and taste-masking effect on the fibrous or dry texture that would otherwise be objectionable.

U.S. Patent 4,232,049 to Blake, issued November 4, 1980, discloses frosting compositions made from a comestible base and minor amounts of whipping agent and polysaccharide gum. The comestible base is prepared by cooking a blend comprising citrus juice vesicles, a nutritive carbohydrate sweetening agent, an ungelatinized starch, an organic acid, and water. Suitable sweetening agents include fructose and dextrose. A preferred optional ingredient is a fruit puree.

U.S. Patent 4,707,374 to King et al., issued November 17, 1987, discloses a cream filling having a water activity of 0.7 or less. The filling is made from high fructose corn syrup which contains dextrose, lactose hydrolysate (glucose and galactose), a modified starch, and a hydrocolloid. The cream is said to be particularly useful in cooked dough products because the low water activity reduces moisture migration from the cream into the dough.

U.S. Patent 4,089,981 to Richardson, issued May 16, 1978, discloses a fibrous food base composition used to make simulated food products such as imitation cheese, imitation ham loaf, or imitation candy loaf. The base composition is made from a mixture of edible oil, water, particulate fibrous cellulose, and gelling agent. Sorbitol, glycerine, dextrose or fructose are added to provide sweetness, humectancy and preserving action.

None of the references discloses or suggests a fiber-containing filling for cookies that has a very low water activity in the range of 0.2 to 0.35, while having a texture that is soft and lubricious instead of tough and fibrous. In particular, none suggests compositions for making such a filling without the need to coat the fiber with fat to provide good taste.

Numerous benefits are obtained by the consumption of fiber. Soluble fiber has been found to have a beneficial blood cholesterol-lowering effect. Insoluble fiber promotes regularity and it is an excellent bulking agent in foods. Both types of fibers provide a reduction in calories because they are substantially nondigestible by the human body.

It is, therefore, an object of the present invention to provide a great-tasting, convenient delivery form for introducing fiber into the diet.

It is another object of the present invention to provide cookies made with fiber-containing fillings.

It is another object of the present invention to provide fillings that have a very low water activity so that the crispness of the outer dough is preserved.

It is another object of the present invention to provide fiber-containing low $A_w$ fillings that are soft and lubricious instead of tough and fibrous.

These and other objects of the present invention will become evident from the disclosures herein.
All parts, percentages and ratios used herein are by weight unless otherwise indicated.


Summary of the Invention


The invention relates to cookies made with a fiber-containing filling. This filling has a very low water activity (0.2-0.35) so that the crispness of the cookies' outer dough is preserved. At the same time, the filling tastes soft and lubricious instead of tough and fibrous. In particular, the invention is a dual textured cookie comprising: (a) discrete regions of a cookie dough, wherein the cookie dough before baking comprises from about 10% to about 30% shortening, from about 20% to about 45% flour, from about 20% to about 45% readily crystallizable sugar, and from about 5% to about 15% water; and (b) discrete regions of a filling, wherein the filling before baking comprises from about 0% to about 25% sorbitol, from about 10% to about 45% fructose, from about 0% to about 30% dextrose, from about 16% to about 32% glycerine, from about 2% to about 35% cellulosic fiber, and from about 3% to about 10% fruit puree.


Detailed Description of the Invention


The present invention relates to shelf stable, dual textured laminated cookies. The cookies provide a great-tasting, convenient delivery form for introducing fiber into the diet. Fiber is added to the cookies as part of a soft, lubricious filling which generally makes up about one-half of the cookies by weight. The filling is surrounded by a crisp textured outer dough. The filling and dough are then baked to make cookies having a taste similar to conventional crisp cookies, but which are much more palatable due to the lubricious, great-tasting filling.

As discussed hereinabove, conventional filling-containing cookies are generally either crisp sandwiched wafers with tough, tacky fillings having low water activity (e.g., 0.20 $A_w$), or soft sandwiched wafers with soft, textured fillings having medium water activity (e.g., 0.55 $A_w$). In contrast, the cookies of the present invention have a crisp outer texture and a soft textured inner filling with a low water activity (0.20-0.35 $A_w$). The filling is shelf stable and has a low enough water activity to preserve the crispness of the basecakes. The combination of low water activity and soft, lubricious texture is achieved by the specific ingredient composition of the fillings of the invention.

In particular, a cookie filling according to the present invention comprises from about 16% to about 32% glycerine, from about 0% to about 25% sorbitol, from about 10% to about 45% fructose, from about 0% to about 30% dextrose, from about 2% to about 35% cellulosic fiber, and from about 3% to about 10% fruit puree. The filling has a water activity between about 0.2 and about 0.35 after baking, and preferably between about 0.2 and about 0.3.

The glycerine is critical for its action as a humectant in the present filling, and it also contributes sweetness. Preferably the filling comprises from about 20% to about 28% glycerine. Although less preferred, it is also possible to replace at least part (or all) of the glycerine with an equal amount of propylene glycol.

The fructose, dextrose and sorbitol also provide sweetness to the filling. The use of dextrose and sorbitol is not critical for making a filling according to the invention, but the filling preferably comprises from about 10% to about 30% dextrose and from about 10% to about 25% sorbitol. On the other hand, it is critical to have at least about 10% fructose in the present filling to achieve the desired taste and texture. Preferably the filling comprises from about 10% to about 25% fructose.

The combination of these ingredients with the other filling ingredients is critical for providing the soft, lubricious texture of the present filling along with its low water activity.

From about 2% to about 35% cellulosic fiber can be incorporated into the present filling, preferably from about 5% to about 25%. By "cellulosic fiber," as used herein, is meant a dietary fiber, or collection of dietary fiber sources, comprised of at least about 20% cellulose or modified cellulosic material (for example, carboxymethyl cellulose). The fiber can contain other fibrous components, such as hemicelluloses, pectins, and lignin. Sources of the cellulosic fiber include vegetables, fruits, seeds, cereals, man-made fibers (for example, by bacterial synthesis), and blends of these fibers. Commercial fibers such as purified plant cellulose, or cellulose flour, can also be used.

Soluble, insoluble, and mixtures of soluble and insoluble fibers can be used in the present invention, depending on the desired benefit. U.S. Patent 4,698,232, at columns 1-2 (incorporated by reference herein) defines and gives examples of soluble and insoluble fibers. For best texture and maximum bulking and regularity benefits, in one preferred embodiment of the present invention the cellulosic fiber is at least about 90% insoluble fiber. Particularly preferred is oat fiber that has been processed to remove some of the soluble fiber, leaving at least about 90% insoluble fiber remaining, for example, Snowite Oat Fiber, manufactured by Canadian Harvest, 2 Barrie Blvd., St. Thomas, Ontario, Canada, N5P 4B9.

For good cholesterol lowering along with the other benefits, in a second preferred embodiment of the invention the fiber is from about 40% to about 60% soluble fiber and from about 40% to about 60% insoluble fiber. A preferred example is fiber made from comminuted citrus peels.

Another preferred fiber for use in the present invention is a low moisture apple fiber (about 5-9% moisture), particularly one that contains about 38% crude fiber, about 55-65% dietary fiber, and about 17% soluble fiber (e.g., Apple Fiber/Bran, manufactured by Canadian Harvest).

The amount of fiber used will be dependent on its absorbency. One skilled in the art will recognize this and adjust the formulation for the water absorbency of the fiber. In general, fibers having low absorbency are preferred so that high fiber levels can be utilized in the filling. For fiber that absorbs between about 2.5 and about 3.5 grams water per gram of fiber, preferably from about 10% to about 20% fiber is used.

For purposes of this invention, absorbency is measured by the following procedure. A standard heat-sealed tea bag is used, made with tea bag filter paper, Dexter Grade 1234 Tea, and measuring 3″ by 2-7/16″. One-half gram of fiber is placed into an empty tea bag. The bag is immersed for 2 minutes in distilled water at room temperature, then removed and hung in the air, and allowed to drip dry for 10 minutes. The bag is then weighed. The procedure is repeated using an empty tea bag without fiber. The weight of the bag alone is subtracted from the weight of the bag containing fiber. This number is divided by the weight of the sample, to obtain an absorbency value measured as grams water/grams fiber.

The fiber also preferably has a particle size such that at least about 80% of the fiber particles have a diameter less than about 300 microns, and preferably less than about 200 microns. Larger particles are detected as being gritty during mastication and are objectionable. Smaller particles are easier to disperse in the product.

The filling additionally comprises from about 3% to about 10% fruit puree. Suitable fruit purees can be derived from apples, pears, grapes, apricots, peaches, strawberries, blueberries, raspberries, and mixtures thereof. The moisture content of the fruit purees should be between about 20% and about 80%, preferably between about 50% and about 75%.

The filling can also comprise from about 0% to about 5% minor ingredients such as acidulants (e.g., citric acid, malic acid, succinic acid), flavors (e.g., fruit flavors), and colors.

The "water activity" ($A_w$) of the cookie filling of the present invention is defined as the partial vapor pressure of water in the filling divided by the saturation vapor pressure of pure water at the same temperature. The water activity of the cookie filling herein can be measured using well known physical chemical techniques and commercially available instruments.

In addition to the fiber-containing filling, the cookies of the present invention also have discrete regions of a cookie dough comprising from about 10% to about 30% shortening, from about 20% to about 45% flour, from about 20% to about 45% readily crystallizable sugar, and from about 5% to about 15% water.

By "shortening" is meant edible fats and oils suitable for cooking or baking, as well as combinations of edible fats and/or oils with appropriate food grade emulsifiers, such as polyglycerol esters, sucrose monoesters, mono- and diglycerides, lecithins, egg yolk and other phospholipids, and the like. Mono-and diglycerides are the preferred emulsifiers. Also included are oleaginous products such as butters and margarines. The shortening can also comprise polyol fatty acid polyesters such as those disclosed in U.S. Patent 3,600,186 to Mattson et al., incorporated by reference herein. Mixtures of triglyceride oils and polyol polyesters can also be used.

By "flour" is meant the finely comminuted meal of any cereal grain or edible seed. Typical non-limiting examples are wheat flour, barley flour, rye flour, corn starch and corn flour, but also encompassed by this term as used herein are the so-called synthetic flours, which incorporate such materials as starch and soy protein isolate, with or without heat and/or steam treatment.

If desired, part of the flour can be replaced by a starch and/or a cellulosic fiber. The cookie dough then comprises from about 15% to about 40% flour, and additionally from about 5% to about 25% dough ingredient selected from starch, cellulosic fiber, or mixtures thereof.

By "readily crystallizable sugar" is meant a mono- or disaccharide, or mixture of mono- and/or disaccharides, that readily and spontaneously crystallizes at the water content and water activity conditions encountered in semi-moist cookies of the home-baked type. Sucrose is virtually unique among the readily available food sugars in this regard, crystallizing spontaneously at $A_w$ levels from about 0.25 to 0.8 in

4

normal cookie systems. Mixtures of readily crystallizable sugars with other mono- and/or disaccharides, where readily crystallizable sugars comprise over 85% of the total sugar, exhibit crystallization behavior similar to a pure readily crystallizable sugar. Hence, readily crystallizable sugars include sucrose and mixtures of sucrose and other mono- and disaccharides which comprise at least 75%, preferably at least 80%, most preferably at least 85% sucrose by weight. For example, fructose and/or molasses are commonly used at low levels in addition to the sucrose.

The cookie dough of the present invention can contain additional flavors and other ingredients depending on the desired kind of cookies, for example, sugar cookies, oatmeal cookies, peanut butter cookies, chocolate chip cookies, and double chocolate chip cookies. For example, oatmeal cookies generally contain rolled oats to provide their characteristic flavor and texture. Peanut butter cookies will, of course, contain peanut butter, which provides not only the distinctive flavor of peanut butter, but also oils (shortening) and peanut solids which supply both carbohydrates and proteins, similar to flour. Other ingredients commonly used include salt, lecithin, glycerine, egg yolk solids, and egg white solids.

If desired, a leavening can be used in the cookie dough. By "leavening" is meant a mixture of a baking soda and a leavening acid. By "baking soda" is meant one of, or a combination of, the common sodas used in baking, such as sodium bicarbonate, sodium carbonate, ammonium bicarbonate, ammonium carbonate, potassium bicarbonate and potassium carbonate. Any of the conventional leavening acids can be used herein such as monocalcium phosphate monohydrate, sodium acid pyrophosphate, sodium aluminum pyrophosphate, sodium aluminum sulfate, and potassium acid tartrate.

Within limits, well known to the art, materials which "interrupt" the homogenous composition of the typical cookie can be introduced into the cookie dough. These materials are essentially inert, so far as the chemistry of the cookie dough is concerned. Examples of such materials, referred to hereinafter as "inclusions", are chopped nuts, chocolate chips or Toll House™ morsels, coconut, butterscotch chips, oatmeal, other cereals, peanut butter chips, raisins, and the like. It may be desirable to incorporate additional flavoring materials, such as spices (e.g., cinnamon, vanilla), cocoa, and the numerous other similar materials commonly found in cookies.

The cookies of the present invention typically comprise between about 30-70% cookie dough and about 30-70% filling, preferably between about 40-60% dough and about 40-60% filling.

A preferred embodiment of the dual-textured cookies of this invention comprises cookies wherein the fiber-containing filling is an inner filling which is completely surrounded and substantially enveloped by an outer crisp dough, thereby forming a ready-to-bake, laminated dough structure. However, the cookies are not limited to this embodiment. Laminated dough structures may be formed by a variety of techniques, such as by applying a layer of crisp cookie dough to only the top part of a mass of filling; by embedding particles or granules of crisp cookie dough in a body of filling, or vice versa; by winding or otherwise distributing strands of extruded crisp cookie dough upon the surface of a ball of filling; by laminating alternating sheets of crisp dough and filling and rolling and slicing to form a "pinwheel" structure; and a variety of other techniques well within the grasp of those in the food production art.

After the cookie dough and filling of the present invention are formed into a laminated structure, they are baked by any standard method to make the finished cookies. By "baking" herein is meant radiant, conductive, dielectric or convective exposure to energy of a type which imparts thermal energy to the product being baked. It thus includes conventional, convection, dielectric and microwave oven baking. Cookies are baked at various times and temperatures; lower baking temperatures in general require longer baking times, and vice versa. One skilled in the art can adjust the baking conditions to meet specific product requirements. Baking can either be performed in the batch mode, as is typically done in the home, or in continuous fashion, as is often done in commercial bakeries.

The following examples illustrate the broad range of industrial applicability of the present invention, without intending to be limiting thereof. It will be appreciated that other modifications of the present invention, within the skill of those in the baking arts, can be undertaken without departing from the spirit and scope of this invention.

Example 1

A batch of dual-textured cookies having a fiber-containing filling is prepared as follows:

5

| Ingredients | Weight Percent |
|---|---|
| **Outer Cookie Dough** | |
| Shortening | 22.9 |
| Fructose | 1.3 |
| Water | 5.5 |
| Molasses | 0.6 |
| Baking soda | 0.5 |
| Sugar (sucrose) | 31.5 |
| Lecithin | 0.1 |
| Flour | 35.6 |
| Glycerine | 2.0 |
| Butter flavor | 0.04 |
| Vanilla flavor | 0.02 |
| **Inner Filling** | |
| Sorbitol | 17.1 |
| Dextrose | 17.1 |
| Fructose | 20.5 |
| Glycerine | 23.9 |
| Apple fiber* | 14.0 |
| Apple puree | 6.8 |
| Citric acid | 0.3 |
| Malic acid | 0.3 |
| Apple flavor | 0.1 |

* Low moisture apple fiber (5-9% moisture), 38% crude fiber, 55-65% dietary fiber, 17% soluble fiber, manufactured by Canadian Harvest, 2 Barrie Blvd., St. Thomas, Ontario, Canada N5P 4B9.

The outer cookie dough is made by first mixing the water with the molasses and soda. The shortening is then added and the ingredients are mixed for 3 to 4 minutes. The sugar is then added and the ingredients mixed again. All the remaining ingredients are then mixed in.

The inner filling is made by first blending together the sorbitol, fructose and dextrose, then adding the glycerine and heating to about 250°F (121°C). This heating sets the water content and water activity of the mixture. The mixture is allowed to cool to about 180°F (82°C), the apple fiber is added, and the ingredients are blended until homogenous. The apple puree, citric acid, malic acid and apple flavor are added, and the ingredients are again blended until homogenous. The filling is allowed to cool to room temperature.

On an industrial scale, the dough and filling are coextruded with a noncommercial extruder to produce a two-dough, concentrically arranged rope. An equivalent coextruder can be employed such as a Rheon[R] encrusting machine, Model 207 available from Rheon Automotive Machinery Company of Japan, or coextruder Model No. DDP 200-9005, available from Bepex Hutt GmbH, Postfach 9, Daimlerstrasse 9, D-7105, Leingarten, West Germany. Then doughballs are formed weighing about 13.4 grams each, and consisting of about 55% cookie dough and about 45% filling. The doughballs are placed onto stainless steel trays and baked in a Middleby-Marshall Oven (Model JS250) at 305°F (152°C) for about 7.5 minutes to make cookies. The cookies are cooled, packaged, and stored.

On a batch scale, the cookie dough and filling are prepared as described above. About 7.5 grams of cookie dough and about 6 grams of filling are used to make a single cookie. Half of the dough is placed and flattened onto a cookie sheet, the filling is placed on top of the dough, and then the remaining dough is flattened and placed on top of the filling. The edges of the dough pieces are brought together so that the filling is enveloped by dough. A batch of cookies is baked in a standard pizza oven at 375°F (191°C) for 7 minutes, then cooled.

The filling has a water activity of about 0.27 after baking. After storage, the outer cookie dough remains crisp while the inner filling is soft, lubricious and good-tasting.

## Example 2

Cookies are made as in Example 1, except that the filling is made with 28% apple fiber instead of 14%, and the percentages of the remaining ingredients are proportionately lowered. After the cookies are baked and cooled, the filling tastes tough and rubbery instead of soft and lubricious.

## Example 3

Cookies are made as in Example 1, except that corn syrup is used in the filling instead of glycerine. The water activity of the filling after baking is about 0.55. As a result of the higher $A_w$, the outer cookie dough becomes soft instead of retaining its crispness.

**Claims**

1. A dual textured cookie comprising:
   (a) discrete regions of a cookie dough, wherein the cookie dough before baking comprises from 10% to 30% shortening, from 20% to 45% flour, from 20% to 45% readily crystallizable sugar, and from 5% to 15% water; and
   (b) discrete regions of a filling, wherein the filling before baking comprises from 0% to 25% sorbitol, from 10% to 45% fructose, from 0% to 30% dextrose, from 16% to 32% glycerine, from 2% to 35% cellulosic fiber, and from 3% to 10% fruit puree, and wherein the filling has a water activity between 0.2 and 0.35 after baking.

2. A cookie according to Claim 1 wherein the filling comprises from 5% to 25% cellulosic fiber.

3. A cookie according to Claim 1 wherein the fiber comprises at least 90% insoluble fiber.

4. A cookie according to Claim 1 wherein the fiber comprises from 40% to 60% soluble fiber and from 40% to 60% insoluble fiber.

5. A cookie according to Claim 1 wherein the dough is an outer dough and the filling is an inner filling.

6. A cookie according to Claim 1 wherein the filling has a water activity between 0.2 and 0.3 after baking.

7. A cookie according to Claim 1 wherein the filling comprises from 10% to 25% fructose.

8. A cookie according to Claim 1 wherein the cookie dough comprises from 15% to 40% flour, and additionally comprises from 5% to 25% dough ingredient selected from the group consisting of starch, cellulosic fiber, and mixtures thereof.

9. A cookie according to Claim 1 wherein the filling comprises from 10% to 25% sorbitol.

10. A cookie according to Claim 1 wherein the filling comprises from 10% to 30% dextrose.